(19) Europäisches Patentamt
European Patent Office
Office européen des brevets

(11) Publication number: 0 323 692 B1

# (12) EUROPEAN PATENT SPECIFICATION

(45) Date of publication of patent specification: 22.06.94

(51) Int. Cl.5: G01N 33/546, //G01N33/94

(21) Application number: 88307172.2

(22) Date of filing: 03.08.88

## (54) Water-insoluble reagent, elements containing same and methods of use.

(30) Priority: 03.08.87 US 81206

(43) Date of publication of application:
12.07.89 Bulletin 89/28

(45) Publication of the grant of the patent:
22.06.94 Bulletin 94/25

(84) Designated Contracting States:
CH DE FR GB LI

(56) References cited:
US-A- 4 161 407
US-A- 4 259 313
US-A- 4 283 382
US-A- 4 430 436
US-A- 4 486 530
US-A- 4 548 870

(73) Proprietor: EASTMAN KODAK COMPANY (a New Jersey corporation)
343 State Street
Rochester New York 14650(US)

(72) Inventor: Sutton, Richard Calvin, EASTMAN KODAK Co.
Patent Dept.,
343 State Street
Rochester, NY 14650(US)
Inventor: Danielson, Susan Jean, EASTMAN KODAK Co.
Patent Dept.,
343 State Street
Rochester, NY 14650(US)

(74) Representative: Nunney, Ronald Frederick Adolphe et al
Kodak Limited
Patent Department
Headstone Drive
Harrow Middlesex HA1 4TY (GB)

EP 0 323 692 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to a water-insoluble reagent which is useful in various immunological methods. It also relates to elements containing the reagent and to immunological methods using same.

Immunoassays, which take advantage of natural immunological reactions, have found widespread use as analytical techniques in clinical chemistry. Because of the specificity of the reactions, they are particularly advantageous in quantifying biological analytes which are present in very low concentration and cannot be adequately quantitated by chemical techniques. Such analytes (called ligands herein) include, for example, therapeutic drugs, narcotics, antibiotics, hormones, proteins, and others known in the art. Several techniques have been devised for determining very low concentrations of ligands. For instance, a ligand may be labeled by various means to make it readily detectable. In competitive binding assays, a labeled ligand analog (identified as ligand analog herein) is placed in competition with unlabeled ligand for reaction with a fixed amount of the appropriate binding material (also called a receptor). Unknown concentrations of the ligand can be determined from the measured signal of either the bound or unbound (that is, free) ligand analog.

Sensitivity is of prime importance due to the extremely low levels of ligands to be determined. While a variety of labels can be used, fluorescent or enzyme labels are generally preferred in most immunoassays due to increased sensitivity.

Immunoassays can also be classified as either heterogeneous or homogeneous. Heterogeneous competitive binding immunoassays require a separation of bound ligand analog from free ligand analog. This separation is necessary because the properties of bound and free analog are not significantly different. Homogeneous immunoassays do not require a separation step because the properties of the bound and free analogs are sufficiently different so that they can be readily differentiated.

Biologically active polypeptides or proteins which are attached to insoluble carrier materials, such as polymeric particles, have been used in a variety of ways in immunoassays. For example, the diagnosis of pathological or other conditions in human beings and animals is often carried out using immunological principles for the detection of an immunologically reactive species, for example antibodies or an antigen, in the body fluids of the person or animal. An antigen is generally known as a foreign substance, such as a drug, hapten, toxin, lectin, glycoprotein, polysaccharide, glycolipid, polypeptide or protein which, when introduced into the body, causes the production of certain soluble proteins known as antibodies.

Other proteins, such as enzymes, have been covalently linked to various carrier materials for use in affinity chromatography, enzymatic reactions, specific binding reactions and immunoassays. Among useful carrier materials are sheep and human erythrocytes, bacterial cells, latex particles, resinous particles and finely divided diazotized amino cellulose. For example, carrier particles prepared from sparingly water-soluble monomers (such as epoxy group-containing monomers) in the absence of emulsifiers are known in the art.

Carboxylated latex particles have also been used to prepare diagnostic reagents. The conventional procedure for covalently attaching an immunologically reactive species to the particles having surface carboxyl groups involves the use of a water-soluble carbodiimide in an additional activation step. While producing useful reagents, this procedure tends to activate the exposed reactive groups of the reactive species as well as the carboxyl groups. The result is intramolecular and intermolecular crosslinking or polymerization of the immunologically reactive species, and a significant portion of the species is thus impaired from complexation with a receptor molecule. Because the reactive species, for example an antibody, is usually very costly, this problem represents a serious economic loss. Further, sensitivity of the resulting reagent may be impaired. It has also been evident that carbodiimides provide a reactive intermediate for protein attachment which is unstable and must be used immediately. This can be a serious drawback to carbodiimide chemistry.

Various other reagents have been prepared with particles having reactive groups such as epoxides, aldehydes, chloromethyl groups, amine groups and diazonium salts. All of these groups have their disadvantages. For example, epoxide groups are not stable so that the particles cannot be stored for very long. Particles having aldehyde groups generally tend to agglutinate prematurely. Particles with amine groups are like the carboxylated materials by requiring an additional activation step. Diazonium compounds are unstable and therefore undesirable to work with.

U.S. Patents 4,283,382 and 4,259,313 describe immunoreactive reagents having europium chelate tracer materials within the particles. Some of the reagents are prepared from polymers having reactive chloromethyl groups. While such materials provide some advantages, attachment of the immunological species to such polymers requires elevated temperatures, extended reaction time and acute mixing conditions. If the attachment conditions are not just right, attachment is incomplete, resulting in a reagent

with poor sensitivity.

Reagents which are composed of a protein attached to a water-insoluble particle are very useful in a number of methods, including immunoassays, diagnostic methods and the like. It would be very useful if highly sensitive reagents could be readily prepared in an efficient manner and under conditions which are not limiting and which do not reduce sensitivity or generate other undesirable results.

The problems noted above with known reagents are overcome with a surfactant-free reagent composed of:

(a) a surfactant-free polymeric particle covalently attached through reactive pendant groups to

(b) an immunological species which is capable of participating in an immunological reaction with a corresponding receptor,

the reagent characterized wherein the polymer is derived from at least one $\alpha,\beta$-ethylenically unsaturated polymerizable monomer having either pendant activated 2-substituted ethylsulfonyl or vinylsulfonyl groups,

the interior of the particle being free of detectable tracer material and the polymer particle having been prepared by emulsion polymerization in the absence of a surfactant or emulsifier.

An element comprises an absorbent carrier material having one or more zones, the element characterized as containing in one or more of the zones a reagent as described above.

Further, a method for the determination of an immunological ligand in an aqueous fluid comprises:

A. in the presence of an analog of the ligand, contacting a sample of the fluid with a reagent as described above,

to form an immunological complex between the immunological species and both the immunological ligand and ligand analog, and

B. determining the immunological complex as an indication of the presence of the ligand in the fluid.

An agglutination method for the determination of a ligand in an aqueous liquid comprises:

A. contacting the liquid with a reagent as described above,

so as to form an agglutinate of the reaction product of the ligand and the immunological species,

B. separating the agglutinate from unagglutinated materials, and

C. determining the agglutinate as an indication of the presence of ligand in the liquid.

A method for the determination of a ligand in an aqueous liquid comprises:

A. contacting the liquid with a reagent as described above,

so as to form an insoluble immunological complex between the immunological species and the ligand,

B. prior to, simultaneously with, or subsequent to contacting step A, contacting the ligand with a second immunological species which is immunologically reactive with the ligand but which is not immunologically reactive with the first immunological species, the second species being labeled with a detectable tracer material,

so as to form a labeled insoluble complex, and

C. determining the labeled insoluble complex as an indication of the presence of the ligand in the liquid.

The present invention provides highly sensitive reagents which can be used in a wide variety of immunological techniques. The polymer particles used to prepare the reagents have readily available functional groups which readily react with proteins, carbohydrates and other biological compounds which have free reactive amine or sulfhydryl groups. The reaction between the functional groups and the proteins or biological compounds can be rapidly carried out under mild pH conditions, low temperatures, and the agglutination, desensitization and instability of known reagents are avoided. The conditions of attachment are not as critical, that is, lower temperatures, shorter times and flexible mixing conditions can be employed without sacrificing sensitivity.

These advantages are achieved in this invention by making the polymeric particles out of ethylenically unsaturated polymerizable monomers that have reactive pendant activated 2-substituted ethylsulfonyl or vinylsulfonyl groups. These groups remain available for reaction after polymerization and enable efficient attachment of immunological species having reactive amine or sulfhydryl groups.

The reagent of the present invention can be used in many different immunoassays wherein the analyte (that is, the ligand) is an immunologically reactive species which has specific binding affinity for the immunological species of the reagent.

The reagent has an immunological species attached to the polymer particle. This species is a component of physiological fluids, cell and tissue extracts or a chemical compound which has at least one reactive amine or sulfhydryl group and is capable of participating in an immunological reaction with a corresponding receptor compound (natural or synthetic) which is a chemical or biological compound which has a reactive site for immunological reaction with the immunological species. By immunological species is

meant either: (1) any substance which, when presented to an immunocompetent host, will result in the production of a specific antibody capable of binding with that substance, or (2) the antibody so produced, which species participates in an antigen-antibody reaction in the use thereof.

Representative immunological species include primary amines, amino acids, peptides, proteins, lipoproteins, glycoproteins, sterines, steroids, lipids, nucleic acids, hormones, vitamins, polysaccharides, carbohydrates, glycolipids, alkaloids, organisms (bacteria, protozoa, fungi, viruses, retroviruses, rickettsia and the like) and components thereof, blood substances, tissue and organ antigens and other materials known to one skilled in the art (see U.S. Patent 4,181,636). In some instances, the immunological species is an antibody which is directed against a hapten, drug, hormone, antibiotic or antigenic material, such as a protein, carbohydrate, polypeptide, glycoprotein, polysaccharide, glycolipid and the like (for example from an organism such as a virus, chlamydial, gonococcal, Streptococcal or similar organism). Alternatively, the immunological species can be an antigen of some type (that is, a polypeptide, carbohydrate, or proteinaceous material) which is immunologically reactive with an antibody. In still other embodiments, the immunological species is an antibody which is directed against another antibody (that is, an anti-antibody). Both monoclonal and polyclonal antibodies can be used, and they can be whole molecules or various fragments thereof, as long as they have at least one reactive amine or sulfhydryl group which can be reacted with the pendant reactive groups of the polymeric particles.

The water-insoluble reagent of the present invention is prepared by attaching the immunological species described above to a water-insoluble polymeric particle of specific composition. These particles are prepared from the $\alpha,\beta$-ethylenically unsaturated polymerizable monomers described below such that there are pendant (that is, free and capable of reaction) activated 2-substituted ethylsulfonyl or vinylsulfonyl groups on the surface of the particles. In some embodiments, the entire particle may be composed of the same polymer. That is, they are homogenous. In other embodiments, however, the particles can be what are known in the art as core-shell polymer particles having a core of a first polymer and a shell of a second polymer, as described in U.S. Patent 4,401,765. In this embodiment, the composition of the core is not critical, but the shell must be composed of the monomers described herein having the requisite pendant reactive groups. In still other embodiments, the particle can be composed of a first polymer onto which is grafted a second polymer which has the requisite pendant reactive groups (see U.S. Patent 3,700,609).

The polymeric particles are generally water-insoluble latex particles having a particle size greater than 0.01 micrometers, preferably in the range of from 0.01 to 5 micrometers, and more preferably from 0.3 to 3 micrometers.

As described above, the polymeric particles useful in the practice of this invention are composed of a polymer derived from at least one $\alpha,\beta$-ethylenically unsaturated polymerizable monomer having either pendant activated 2-substituted ethylsulfonyl or vinylsulfonyl groups. A number of representative monomers having the requisite pendant groups are known in the art, including U.S. Patents 4,161,407 and 4,548,870.

Specifically useful polymers are those represented by the formula:

$$-(A)_x-(B)_y-(D)_z-$$

wherein A represents recurring units derived from one or more hydrophobic ethylenically unsaturated monomers. Such monomers are insoluble in water. Representative hydrophobic monomers include, but are not limited to, styrene and styrene derivatives (for example, vinyltoluene, 2,5-dimethylstyrene, 4-*t*-butylstyrene and 2-chlorostyrene), acrylic and methacrylic acid esters (for example, *n*-butyl acrylate, propyl methacrylate, methyl acrylate, ethyl methacrylate, 2-ethylhexyl methacrylate and methyl methacrylate) and vinyl acetate.

The polymer useful in this invention can be crosslinked, if desired, in any suitable fashion. One method is to incorporate a small amount, that is up to 15 mole percent, and preferably from 0.3 to 5 mole percent, of a monomer having two or more ethylenically unsaturated polymerizable groups. These monomers are included among the hydrophobic monomers from which A is derived. Representative monomers are described in *Research Disclosure*, publication 19551, July, 1980, page 304, and include for example, divinylbenzene, ethylene dimethacrylate, N,N'-methylenebisacrylamide, 2,2-dimethyl-1,3-propylene diacrylate, allyl acrylate, ethylidyne trimethacrylate and ethylene diacrylate.

Particularly useful monomers from which A is derived are styrene, vinyltoluene, ethylene dimethacrylate, butyl acrylate, divinylbenzene, 2-ethylhexyl methacrylate and methyl methacrylate.

B represents recurring units derived from one or more $\alpha,\beta$-ethylenically unsaturated monomers represented by the formula:

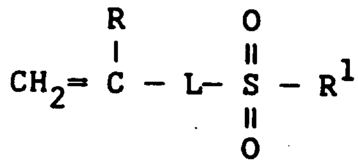

wherein R is hydrogen or substituted or unsubstituted alkyl (generally of 1 to 6 carbon atoms, such as methyl, ethyl, isopropyl or hexyl. Preferably, R is hydrogen or methyl.

$R^1$ is $-CH=CHR^2$ or $-CH_2CH_2X$ wherein X is a leaving group which is displaced by a nucleophile or is eliminated in the form of HX by treatment with a base (such as halo, acetoxy, alkylsulfonyloxy such as methylsulfonyloxy, arylsulfonyloxy such as p-tolylsulfonyloxy, trialkylammonio, for example, a trimethylammonio salt or pyridinio salt). $R^2$ is hydrogen, substituted or unsubstituted alkyl (generally of 1 to 6 carbon atoms as defined for R), or substituted or unsubstituted aryl (generally of 6 to 12 nuclear carbon atoms, such as phenyl, naphthyl, xylyl or tolyl). Preferably, $R^1$ is $-CH_2CH_2X$. This group, which is an activated 2-substituted ethyl group, can be substituted with any group which does not impair the displacement of the leaving group X.

L is a linking group which can be a substituted or unsubstituted alkylene generally having 1 to 20 atoms selected from carbon and hetero atoms in the backbone. This definition of alkylene is meant to include alkylene groups interrupted or terminated with oxy, thio, $-NR^3-$ [wherein $R^3$ is hydrogen, substituted or unsubstituted alkyl of 1 to 6 carbon atoms (such as methyl, chloromethyl or 2-hydroxyethyl) or substituted or unsubstituted aryl of 6 to 10 carbon atoms (such as phenyl, naphthyl or xylyl)], ester (-COO-), amide (-CONH-), urylene

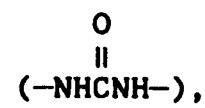

sulfonyl ($-SO_2-$), carbonate, sulfonamide, azo, phosphono or other similar groups. Representative alkylene groups include methylene, ethylene, isobutylene, hexamethylene, carbonyloxyethoxycarbonyl, methylenebis(iminocarbonyl), carbonyloxydodecylenecarbonyloxyethylene, carbonyliminomethyleneiminocarbonyliminoethylene, carbonyliminomethyleneiminocarbonylethylene and other groups described or suggested by U.S. Patents 4,161,407 and 4,548,870.

L can also be substituted or unsubstituted arylene generally having 6 to 12 nuclear carbon atoms. Representative arylene groups include phenylene, tolylene, naphthylene and others noted in the patents mentioned above. Also included in this definition of L are divalent groups which are combinations of one or more of each of the alkylene and arylene groups defined above (for example, arylenealkylene, alkylenearylenealkylene and others readily determined by one of ordinary skill in the art). Preferably, L is substituted or unsubstituted phenylenealkylene, phenylenealkylene substituted with one or more alkyl groups (as defined for R), alkoxy groups (generally of 1 to 6 carbon atoms, for example, methoxy, propoxy or butoxy) or halo groups, or carbonyliminomethyleneiminocarbonylethylene.

Representative monomers from which B can be derived include m & p-(2-chloroethylsulfonylmethyl)-styrene, m & p-[2-(p-tolylsulfonyloxy)ethylsulfonylmethyl]styrene, m & p-vinylsulfonylmethylstyrene, N-[m & p-(2-chloroethylsulfonylmethyl)phenyl]acrylamide, and N-[2-(2-chloroethylsulfonyl)ethylformamidomethyl]-acrylamide. The first monomer is preferred.

D represents recurring units derived from one or more ethylenically unsaturated monomers other than those represented by A or B. Generally such monomers have ionic or other hydrophilic groups which add dispersion stability to the resulting particles in aqueous solution. Useful ionic monomers include, but are not limited to, sodium 2-acrylamido-2-methylpropanesulfonate, sodium 3-acryloyloxypropanesulfonate, sodium acrylate, sodium methacrylate, and sodium styrenesulfonate, as well as other known sulfonates, sulfates, carboxylates, their salts or anhydrides, and useful nonionic polar monomers include 2-hydroxyethyl acrylate, 2,3-dihydroxypropyl acrylate, acrylamide, 2-hydroxyethyl methacrylate, N-isopropylacrylamide, 2-hydroxypropyl methacrylate, acrylonitrile and N-isbutoxymethyl acrylamide. Preferred monomers are sodium 2-acrylamido-2-methylpropanesulfonate, sodium acrylate, sodium 3-acryloyloxypropanesulfonate, sodium methacrylate, 2-hydroxyethyl acrylate, 2,3-dihydroxypropyl acrylate, acrylamide, N-isopropylacrylamide and acrylonitrile.

In the formula defined above, generally, x is from 0 to 99.9 mole percent, y is from 0.1 to 100 mole percent, and z is from 0 to 20 mole percent. Preferred amounts are from 50 to 99.5 mole percent of x, from 0.5 to 50 mole percent of y and from 0 to 10 mole percent of z.

Representative polymers of which the particles are composed include:

poly[styrene-co-m & p-chloroethylsulfonylmethyl styrene] (99.5:4.5 molar ratio), and

poly{styrene-co-N-[m & p-(2-chloroethylsulfonylmethyl)phenyl]acrylamide} (99.3:0.7 molar ratio). The first polymer is preferred. Further details relating to these polymers and representative materials can be found in U.S. Patents 4,161,407 and 4,548,870.

The polymeric particles can be prepared using emulsion (including batch, semi-continuous and continuous) techniques without the use of surfactants or emulsifiers as described for example in U.S. Patent 4,415,700 and Research Disclosure publication 15963 (July, 1977).

The general procedure for preparing the reagent of this invention by covalently attaching the immunological species to the surfactant-free particles is as follows:

the polymer particles are mixed with the immunological species in an aqueous buffered solution (pH generally from 7 to 10) and a concentration of from 0.01 to 40 weight percent polymer particles (preferably from 0.01 to 10 weight percent). The amount of immunological species is at a ratio of species to polymer of from 0.1:1000 to 1:10, and preferably from 1:100 to 1:10 by weight. Mixing is carried out at a temperature in the range of from 5 to 50 °C, and preferably at from 5 to 25 °C, for from 0.5 to 48 hours. Any suitable buffer can be used, but a tertiary amine is preferred. The details of this procedure are illustrated in Example 1 below.

The polymeric particles used in this invention contain no detectable tracer material within the particles. In the uses of this reagent, a tracer inside the particle would either be disadvantageous, unneeded where an external tracer is used, or unneeded where detecting the reagent is irrelevant to the use. A tracer is a material which is detectable using appropriate equipment and techniques. This means that the interior of the particles contain no detectable amounts of tracer materials, such as colorimetric or fluorometric dyes or compounds (such as rare earth chelates), chemiluminescent compounds, phosphorescent compounds, radioisotopes, or bioluminescent compounds. In some embodiments, the reagent can have a tracer material (such as an enzyme or radioisotope) associated therewith on the particle surface or somehow attached to the immunological species. In other embodiments, the reagent has no tracer associated with it in any manner.

The reagent of the present invention can be used in the determination (qualitative or quantitative measurement) of an analyte in aqueous liquids. This determination can be made by merely determining the presence or absence of the analyte, or by quantitatively determining the amount of analyte. Where the analyte is determinable by immunological methods, it is identified as a ligand herein. In particular, the invention can be used to assay biological fluids of animals, humans or plants, but preferably of humans. Such fluids include, but are not limited to, whole blood, plasma, sera, lymph, bile, urine, spinal fluid, sputum, perspiration and the like as well as stool secretions. It is also possible to assay fluid preparations of human or animal tissue such as skeletal muscle, heart, kidney, lungs, brains, bone marrow, skin and the like.

The present invention can be used to detect or quantify any of a wide variety of ligands which are reactive with the immunological species on the reagent of the invention. Such ligands include, but are not limited to, proteins, hormones, drugs, haptens, carbohydrates, plant lectins or lipopolysaccharides which have one or more sites for complexing with the immunological species of the reagent. For example, the reagent comprises antibodies directed against the ligand which may be a drug or hapten. The invention can be particularly useful in the determination of digoxin, phenytoin, phenobarbital, thyroxine, triiodothyronine, gentamicin, carbamazepine, Primidone, tobramycin or theophylline.

Alternatively, the ligand can be an antibody which has two or more sites for complexation with one or more immunological species, one of which is part of the reagent of this invention. In diagnostic assays described herein, the ligand can be polypeptides, enzymes, fungi, protozoa, Streptococcus A antigen, antigens from chlamydial or gonococcal organisms, a retroviral antigen or antibody (such as HTLV antigens or antibodies or HIV antigens or antibodies), thyroid stimulating hormone, apolipoproteins, human chorionic gonadotropin, leutinizing hormone, herpes viruses and other biological compounds, organisms or components thereof.

The reagent can be used in a solution assay method in competitive binding immunoassays. By solution assay is meant that the reagents of this invention are used in liquid suspension in an immunoassay. Either bound (that is, complexed) or unbound (that is, uncomplexed) labeled materials can be determined. Physical separation of bound and unbound materials, if desired, can be carried out using any suitable separation technique. In using the analytical elements described below, either vertical or horizontal

separation can be used.

In a competitive binding assay, the reagent is generally present in a concentration in an amount which depends upon the amount of immunological species on the polymeric particles and the type of assay conducted. It also depends upon the binding constant of the immunological species. Suitable amounts for a given assay can be readily determined by one of ordinary skill in the art. The corresponding ligand analog can be present in amounts generally known in the art for a given assay. Other materials, for example, buffers, surfactants, reagents for color formation, can be used in the assay if desired.

An assay is generally carried out by physically contacting and mixing the reagent, the labeled ligand analog and the sample to be tested in a suitable container. The resulting suspension can be incubated, if desired, to promote complexation and other reactions. The sample is then evaluated using suitable detection equipment and procedures.

In another embodiment, the reagent can be used in what are known in the art as immunometric assays, for example, "sandwich" assays. The details of such assays are provided in U.S. Patent 4,486,530. The reagent of the present invention is useful in such assays where the ligand to be determined has two or more epitopic sites for immunological reaction with two or more receptor molecules. The receptor molecules can be the same or different. One of the receptor molecules is identified herein as a first immunological species attached as a part of the reagent of this invention. A second immunological species is also used which is capable of immunologically reacting with the ligand at a site different than the site where the first species react. The result of the method is the formation of a complex of the two distinct immunological species with the ligand. The second species (that is, the one not part of the reagent of this invention) is labeled in some manner so the resulting insoluble complex can be readily detected. In an immunometric assay, both immunological species are distinct antibodies directed against an antigen. They can be the same or different antibodies, whole or fragments, monoclonal or polyclonal.

In still another embodiment of this invention, the reagent of this invention is comprised of an antigen and the ligand to be determined is an antibody. A second immunological species used in the assay is a second antibody which is directed against the first antibody. The second immunological species can be labeled or unlabeled. If the second antibody is unlabeled, a labeled third antibody directed against the second antibody, or a labeled second antigen molecule reactive with the second antibody can be used.

The methods of this invention described above, can also be practiced with a dry analytical element. The simplest element can be composed of an absorbent carrier material, for example, a thin sheet of a self-supporting absorbent or bibulous material, such as filter paper or strips, which has one or more zones, at least one zone containing the reagent of this invention. Other zones can be used to contain other useful reagents. Such elements are known in the art as test strips, diagnostic elements, dip sticks or diagnostic agents.

Useful absorbent carrier materials are insoluble and maintain their structural integrity when exposed to water or biological fluids such as whole blood or serum. Useful elements can be prepared from paper, porous particulate structures, porous polymeric films, cellulose, glass fibers, woven and nonwoven fabrics (synthetic and nonsynthetic) and the like. Useful materials and procedures for making such elements are well known in the art.

Preferably, the absorbent carrier material of the dry analytical element of this invention is a porous spreading zone. This zone can be self-supporting (that is, composed of a material rigid enough to maintain its integrity), but preferably it is carried on a separate support. Such a support can be any suitable dimensionally stable, and preferably, nonporous and transparent (that is, radiation transmissive) material which transmits electromagnetic radiation of a wavelength between 200 and 900 nm. A support of choice for a particular element should be compatible with the intended mode of detection (fluorescence, transmission or reflectance spectroscopy). Useful supports can be prepared from paper, metal foils, polystyrene, polyesters, polycarbonates or cellulose esters.

The porous spreading zone can be prepared from any suitable fibrous or non-fibrous material or mixtures of either or both. The void volume and average pore size of this zone can be varied depending upon the use intended. Useful spreading zones can be prepared using materials and procedures described, for example, in U. S. Patents 4,292,272, 3,992,158, 4,258,001 and 4,430,436 and Japanese Patent Publication 57(1982)-101760.

The elements can have two or more discrete zones, either in the same layer or superimposed. At least one of the zones is preferably a porous spreading zone. The other zones can be reagent zones or registration zones as those zones are known in the art, additional spreading zones, radiation-blocking or filter zones, subbing zones or barrier zones. The zones are generally in fluid contact with each other, meaning that fluids, reagents and reaction products (for example, color dyes) can pass or be transported between superposed regions of adjacent zones. In other words, when the element is contacted with fluid,

the reagents within the element become mixed and can readily interact. Preferably, each zone is a separately coated layer, although two or more zones can be separate areas in a single layer of the element.

While the reagent of this invention is located in the element, it is not critical in a competitive binding assay that the ligand analog also be located therein. It is possible to add the ligand analog to the element at the time of assay. Preferably, however, the ligand analog and the reagent of this invention are both in the element, and isolated from each other in such a manner such that they will not interact prior to the assay. For example, one or both of these materials can be encapsulated with a substance that will dissolve in the applied aqueous sample. Alternatively, they can be isolated by putting them in different zones of the element where they do not mix until the assay is carried out.

In one embodiment for a competitive binding assay, an analytical element for the immunological determination of a drug or hormone, comprises a nonporous support having thereon, in order and in fluid contact,

a reagent layer containing one or more reagents for providing a detectable signal in the determination,

a water-soluble layer containing an enzyme-labeled analog of the drug or hormone, and

a porous spreading layer containing a reagent consisting essentially of:

(a) a polymeric particle composed of a polymer represented by the formula:

$$-(A)_x-(B)_y-(D)_z-$$

wherein A represents recurring units derived from one or more hydrophobic ethylenically unsaturated monomers,

B represents recurring units derived from one or more ethylenically unsaturated monomers represented by the formula:

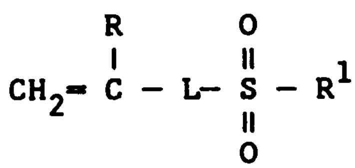

as defined above,

D represents recurring units derived from one or more ethylenically unsaturated monomers other than those represented by A or B,

x is from 0 to 99.9 mole percent, y is from 0.1 to 100 mole percent, and z is from 0 to 20 mole percent,

the interior of the particle being substantially free of detectable tracer material, and

the particle being covalently attached through the B recurring units to

(b) one or more antibody molecules directed against the drug or hormone.

A variety of different elements, depending on the method of assay, can be prepared in accordance with the present invention. Elements can be configured in a variety of forms, including elongated tapes of any desired width, sheets, slides or chips.

The assay of this invention can be manual or automated. In general, in using the dry elements, analyte determination is made by taking the element from a supply roll, chip packet or other source and physically contacting it with a sample (for example, up to 500 µl) of the liquid to be tested so that the sample and reagents within the element become mixed. Such contact can be accomplished in any suitable manner, for example, by dipping or immersing the element into the sample or, preferably, by spotting the element by hand or machine with a drop of the sample with a suitable dispensing means. U.S. Patent 4,670,381 provides additional details of sample application. Wash fluids can also be used, as described, for example, in U.S. Patent 4,517,288.

After sample application, the element can be exposed to conditioning, such as incubation, heating or the like, that may be desirable to quicken or otherwise facilitate obtaining any test result.

In still another embodiment of this invention, the reagent of this invention can be used in agglutination assays to determine the presence of a ligand which forms a complex with the immunological species in an immunological reaction. The resulting complex precipitates in a detectable agglutination or clumping of particles. The agglutination can be detected, for example, visually or with suitable light scattering detection

equipment. Useful agglutination techniques are described in U.S. Patent 4,419,453.

The following preparations illustrate representative methods of preparing polymer particles useful in the practice of this invention.

Preparation 1: Preparation of Poly[styrene-co-m & p(2-chloroethylsulfonylmethyl)styrene] (95.5:4.5 molar ratio)

Three solutions of reagents were simultaneously added and mixed using a continuous emulsion polymerization technique in a vessel at 80 °C. Solution 1 contained styrene (739 g), m & p-(2-chloroethylsulfonylmethyl)styrene (82 g) and 1-dodecanethiol (8.2 g). Solution 2 contained ammonium peroxydisulfate (19.7 g) in distilled water (1152 g). Solution 3 contained sodium pyrosulfite (9.85 g) in distilled water (1152 g).

The solutions were pumped into the vessel at the following individual rates: Solution 1, 2.5 g/min., Solution 2, 2.14 g/min., and Solution 3, 2.27 g/min. After an addition time of 380 minutes, the reaction was stopped, and the yield was 1218 g at 33.4% solids. The polymer latex was then dialyzed for 3 days to yield a latex at 27.3% solids having a pH of 5. This latex was diluted to 13.5% solids for testing. Nuclear magnetic resonance analysis of the polymer indicated a molar ratio of 96:4, styrene to the sulfonyl comonomer.

Preparation 2: Preparation of Core/Shell Polymer Particles Having a Core of Poly(styrene-co-2-acetoacetoxyethyl methacrylate) (85:15 molar ratio) and a Shell of Poly[styrene-co-m & p-(2-chloroethylsulfonylmethyl)-styrene] (95.5:4.5 molar ratio)

A procedure similar to that described in Preparation 1 was followed to make a core/shell polymer. Three solutions were simultaneously added to and mixed in a vessel as follows. Solution 1 contained styrene (179.1 g), 2 acetoacetoxyethyl methacrylate (65 g) and 1-dodecanethiol (2.4 g) and was pumped at 1.5 g.min. Solution 2 contained ammonium peroxydisulfate (8.14 g) in distilled water (828 g), and was pumped at 2.36 g/min. Solution 3 contained sodium pyrosulfite (4.1 g) in distilled water (828 g), and was pumped at 2.44 g/min.

The solutions were pumped into the vessel over a period of 164 minutes. The solids content was 10.3% and the residence time was 213 minutes at 80 °C. The resulting polymer particles were used as the core of the resulting core/shell polymer.

The shell was provided by simultaneously adding the following solutions to the vessel containing the core particles: Solution 4 contained styrene (146.7 g), m & p-(2-chloroethylsulfonylmethyl)styrene (16.3 g) and 1-dodecanethiol (1.6 g) and was pumped at a rate of 1.41 g/min. Solutions 5 and 6 were the same as Solutions 2 and 3, respectively. The addition time for these solutions was 111 minutes at 80 °C. No new particles were formed, but the monomers of Solution 4 polymerized as a copolymer shell on the core particles previously prepared. The resulting core/shell polymer solids content was 13.8% in the solution which was then dialyzed for 4 days.

Example 1: Preparation of Reagent Containing Bovine Gamma Globulin

This example illustrates the preparation of a reagent of the present invention. It also demonstrates the improvement of the present invention over similar reagents prepared according to the prior art.

A portion of the latex prepared as described in Preparation 1 above containing 30 mg dry weight of polymer was combined with 0.3 mg of tritiated bovine gamma globulin protein, and the solution was brought to a final volume of 10 ml with 0.1 molar sodium borate (pH 8.5) in a centrifuge tube. Reaction of the protein with the surface reactive groups of the polymer particles was continued for 24 hours at room temperature (about 25 °C) with end-over-end rotation at 30-35 rpm while attached to a rotating plate mounted at a 45 ° angle. A second portion of the latex was similarly reacted with protein except that the incubation was carried out at 37 °C.

A third portion of latex was similarly reacted with 1.5 g tritiated protein at 25 °C, while a fourth portion was reacted with 1.5 g of protein at 37 °C. In all portions of polymer, the latex particles had an average particle size of 0.68 micrometers.

Four control reagents were prepared by substituting 30 mg of a core-shell polymer having surface chloromethyl reactive groups. The core of this polymer was composed of poly(styrene-co-divinylbenzene) (99.2:0.8 molar ratio), and the shell was composed of poly(vinylbenzyl chloride-co-divinylbenzene) (98.8:1.2 molar ratio). The average particle size was about 0.68 micrometers. The core/shell polymer was divided into

four portions and reacted under the same conditions with tritiated bovine gamma globulin as described for the reagent of this invention.

At the end of the described incubation times, each reaction was quenched by addition of excess bovine serum albumin (30 mg, 30mg/ml of buffer). The reagents were then incubated another 4 hours after this addition.

The total amount of protein bound was determined by measuring: (a) the total counts per minute in a 500 microliter aliquot of the reaction mixture, (b) the counts per minute remaining in the supernatant following centrifugation of a 1 ml sample of the reaction mixture, and (c) the counts per minute from labeled protein bound to the latex following repeated washes of the pellet obtained in (b). The fraction of the labeled protein covalently bound to the particles was determined following incubation of the reacted particles in the presence of 1% sodium dodecylsulfate surfactant at 37°C for about 24 hours with end-over-end rotation. The same procedure described above for determining the total amount of protein bound is used to determine the amount of protein covalently bound. The results are presented in Tables I and II below.

TABLE I

| Reagent (Portion) | Incubation Temperature | Tritiated Protein Used (mg) | % Protein Bound | mg Protein/g/Polymer |
|---|---|---|---|---|
| Example 1 | | | | |
| (1) | 25°C | 0.3 | 94 | 9.4 |
| (2) | 37°C | 0.3 | 93 | 9.3 |
| (3) | 25°C | 1.5 | 86 | 43 |
| (4) | 37°C | 1.5 | 84 | 42 |
| Control | | | | |
| (1) | 25°C | 0.3 | 94 | 9.4 |
| (2) | 37°C | 0.3 | 90 | 9.0 |
| (3) | 25°C | 1.5 | 69 | 34 |
| (4) | 37°C | 1.5 | 83 | 42 |

TABLE II

(After Surfactant Treatment)

| Reagent (Portion) | Incubation Temperature | Tritiated Protein Used (mg) | % Protein Bound | mg Protein/ g/Polymer | Ratio Covalent/Total |
|---|---|---|---|---|---|
| Example 1 | | | | | |
| (1) | 25°C | 0.3 | 71 | 7.1 | 0.76/1 |
| (2) | 37°C | 0.3 | 81 | 8.1 | 0.87/1 |
| (3) | 25°C | 1.5 | 40 | 20 | 0.46/1 |
| (4) | 37°C | 1.5 | 46 | 23 | 0.57/1 |
| Control | | | | | |
| (1) | 25°C | 0.3 | 46 | 4.6 | 0.43/1 |
| (2) | 37°C | 0.3 | 75 | 7.5 | 0.77/1 |
| (3) | 25°C | 1.5 | 19 | 9.6 | 0.20/1 |
| (4) | 37°C | 1.5 | 52 | 26 | 0.55/1 |

This example demonstrates the preparation of a useful reagent in the practice of this invention. This reagent is labeled in the protein portion with a radioisotope. The data presented above indicate that the protein can be covalently attached to the polymeric particles containing reactive chloroethylsulfonyl groups using ambient (that is, room temperature) conditions much more efficiently than the corresponding Control materials. The chloromethyl group of the Control particles requires elevated incubation temperatures to obtain efficient (that is, high amounts of) binding. The advantage of the reagent of this invention is that lower temperatures may be required for attachment with certain immunoreactive species or enzymes which are inactivated at the higher temperatures.

It is also apparent from this example, that the reagent of this invention can be prepared without resort to activation steps or reagents as is common with some prior art procedures.

Examples 2 and 3: Preparation and Use of Reagents in Immunoassay for Phenobarbital

This example demonstrates the preparation of two reagents of the present invention and their use in immunoassays to determine the presence of the drug, phenobarbital.

A monoclonal antibody directed against phenobarbital was prepared in the laboratory at Eastman Kodak Company using standard procedures of immunization of Balb/c mice with phenobarbital-human serum albumin. The spleens of the immunized mice were fused with myoloma cells (SP1/ϕ-Ag14) to generate hybridomas which produced the desired monoclonal antibodies.

In Example 2, this antibody was covalently attached to polymeric particles like those described in Example 1 above containing reactive chloroethylsulfonyl groups. In Example 3, the antibody was covalently attached to polymeric particles composed of poly[styrene-co-N-(p-chloroethylsulfonylmethylphenyl)-acrylamide] (99.27:0.73 molar ratio) which had an average diameter of about 0.83 micrometers. The mass of the antibody bound to the particles was determined in a parallel experiment in which tritiated bovine gamma globulin was used in place of the anti-phenobarbital antibody. The amount of active protein bound to the particles was compared in the enzyme label binding experiment described below.

One sample of the latex particles (30 mg dry weight of polymer) was mixed with 0.3 mg of the anti-phenobarbital antibody in 10 ml of 3-{[tris(hydroxymethyl)methyl]amino}propanesulfonic acid buffer (pH 8.5, 0.1 molar). A second sample of the latex was mixed with 1.5 mg of the antibody in 10 ml of the same buffer. The attachment reactions are carried out by incubation for 24 hours with end-over-end rotation at room temperature. The reactions were stopped by the addition of bovine serum albumin (30 mg, 30 mg/ml), and the incubation was continued for another 4 hours. The reaction mixtures were then centrifuged, the supernatant discarded, and the pellets washed once with phosphate buffered saline solution (pH 7.4) and then resuspended in the saline solution.

One sample (30 mg of dry polymer) of a Control latex like that shown in Example 1 was similarly incubated with the anti-phenobarbital antibody (0.3 mg) in 10 ml of 0.1 molar sodium borate buffer at pH 8.5. A second portion of the latex was similarly incubated with 1.5 mg of the antibody in the same buffer. The attachment reactions were carried out at 37 °C instead of room temperature.

The mass of antibody bound to each latex preparation was determined by assaying the number of counts for samples run in parallel having tritiated bovine gamma globulin bound to the particles as described in Example 1. The covalent/total ratio was calculated as described in Example 1 after incubation with sodium dodecylsulfate surfactant. The relative amount of active antibody in each preparation was determined in an assay in which serial dilutions of the reagent were mixed with a fixed concentration of glucose oxidase-labeled phenobarbital ($5 \times 10^{-10}$ molar). The reagent amounts used varied from $6.3 \times 10^{-10}$ molar to $2.0 \times 10^{-7}$ molar theoretical phenobarbital binding sites based on the mass of antibody bound.

The reagent dilutions and labeled drug were incubated for about 1 hour with constant agitation at room temperature in phosphate buffered saline solution containing 1% bovine serum albumin. The amount of phenobarbital-glucose oxidase label remaining in solution following centrifugation was determined and the concentration of phenobarbital binding sites required to bind 50% of the enzyme label was determined. The results of this experiment are summarized in Tables III and IV below.

TABLE III

| (Mass Binding Experiment) | | | | |
|---|---|---|---|---|
| Reagent | Amount Labeled Protein Used (mg) | % Bound | mg$^3$H Protein/gm Polymer | Covalent/Total |
| Example 2 | 0.3 | 88 | 8.8 | 0.86/l |
| Example 2 | 1.5 | 62 | 31 | 0.73/l |
| Example 3 | 0.3 | 85 | 8.5 | 0.84/l |
| Example 3 | 1.5 | 62 | 30.8 | 0.64/l |
| Control | 0.3 | 88 | 8.8 | 0.83/l |
| Control | 1.5 | 73 | 37 | 0.69/l |

TABLE IV

| (Latex-Enzyme Label Titration) | | |
|---|---|---|
| Reagent | Amount Protein Used (mg) | Theoretical Phenobarbital Binding Sites to Bind 50% of Label (nmolar) |
| Example 2 | 0.3 | 43 |
| Example 2 | 1.5 | 6 |
| Example 3 | 0.3 | 13 |
| Example 3 | 1.5 | 11 |
| Control | 0.3 | 114 |
| Control | 1.5 | 25 |

This example demonstrates that the anti-phenobarbital antibody can be covalently bound to the polymeric particles having reactive chloroethylsulfonyl groups using ambient reaction conditions at very high efficiency. However, the Control reagent must be prepared at elevated temperatures in order to have efficient attachment. It also demonstrates that the antibody can be attached to form the reagent of this invention using mild conditions with a 3 to 4 times greater retention of antibody activity than for the Control reagent.

Example 4: Preparation and Use of Reagent in Immunoassay for Digoxin

A monoclonal antibody directed against digoxin (DAS5) was purchased from Beckman.

This antibody was covalently attached to polymeric particles containing reactive chloroethylsulfonyl groups. The particles were core/shell bead particles having a core of poly(styrene-co-divinylbenzene) (99:1 molar ratio), a shell of poly[styrene-co-m- & p-(2-chloroethylsulfonylmethyl)styrene-co-divinylbenzene] (94.5:4.5:1 molar ratio), and an average diameter of about 0.65 micrometers.

One sample of the latex particles (100 mg dry weight of polymer) was mixed with 3.0 mg of the anti-digoxin antibody in 10 ml of 3-{[tris(hydroxymethyl)methyl]amino}propanesulfonic acid buffer (pH 8.5, 0.1 molar). The attachment reaction was carried out by incubation for 24 hours with end-over-end rotation at room temperature. The reaction was stopped by the addition of bovine serum albumin (100 mg, 50 mg/ml) and the incubation was continued for an additional 4 hours. The reaction mixture was centrifuged, the supernatant discarded, and the pellets washed once with phosphate buffered saline solution (pH 7.4) and then resuspended in the saline solution.

A sample of a control latex, as employed in Example 1 except having an average particle size of 0.79 µm, (100 mg of dry polymer) was similarly incubated with the anti-digoxin antibody (3 mg) in 10 ml of 0.1 molar sodium borate buffer at pH 8.5. The attachment reactions were carried out at 37°C instead of room temperature.

The relative amount of active antibody in each preparation was determined in an assay in which serial dilutions of the reagent were mixed with a fixed concentration of horseradish peroxidase-labeled digoxin ($5 \times 10^{-11}$ molar). The reagent amounts varied from $2.5 \times 10^{-10}$ to $2.5 \times 10^{-4}$ g of beads containing the immobilized antibody.

The reagent dilutions and labeled drug were incubated for about 1 hour with constant agitation at room temperature in phosphate buffered saline containing 0.1% bovine serum albumin. The amount of digoxin-peroxidase label remaining in solution following centrifugation was determined and the concentration of beads required to bind 50% of the enzyme label was determined. The results of this experiment are summarized in Table V below:

TABLE V

| (Latex-Enzyme Label Titration) | | |
|---|---|---|
| | Amount Protein Used (mg) | Beads to Bind 50% of Label (µg) |
| Example 4 | 3 | 0.119 |
| Control | 3 | 0.562 |

This example demonstrates that the anti-digoxin antibody can be attached to form the reagent of this invention which has a five times greater retention of antibody activity than the Control reagent.

## Claims

1. A surfactant-free reagent composed of:
   (a) a surfactant-free polymeric particle covalently attached through reactive pendant groups to
   (b) an immunological species which is capable of participating in an immunological reaction with a corresponding receptor,
   the reagent characterized wherein the polymer is derived from at least one α,β-ethylenically unsaturated polymerizable monomer having either pendant activated 2-substituted ethylsulfonyl or vinylsulfonyl groups,
   the interior of the particle being free of detectable tracer material and the polymer particle having been prepared by emulsion polymerization in the absence of a surfactant or emulsifier.

2. The reagent as claimed in claim 1 wherein the polymer is represented by the formula:

$$-(A)_x-(B)_y-(D)_z-$$

   wherein A represents recurring units derived from one or more hydrophobic α,β-ethylenically unsaturated monomers,
   B represents recurring units derived from one or more α,β-ethylenically unsaturated monomers represented by the formula:

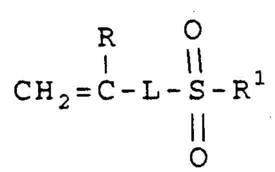

   wherein R is hydrogen or substituted or unsubstituted alkyl of 1 to 6 carbon atoms,
   $R^1$ is $-CH=CHR^2$ or $-CH_2CH_2X$ wherein X is a leaving group which is displaced by a nucleophile or is eliminated in the form of HX by treatment with a base, and $R^2$ is hydrogen, substituted or unsubstituted alkyl of 1 to 6 carbon atoms, or substituted or unsubstituted aryl of 6 to 12 carbon atoms, and
   L is a linking group selected from the group consisting of substituted or unsubstituted alkylene of 1 to 20 carbon atoms, substituted or unsubstituted arylene of 6 to 12 carbon atoms, and a combination of one or more of each of the alkylene and arylene groups,
   D represents recurring units derived from one or more α,β-ethylenically unsaturated monomers other than those represented by A or B,
   x is from 0 to 99.9 mole percent, y is from 0.1 to 100 mole percent, and z is from 0 to 20 mole percent.

3. The reagent as claimed in claim 2 wherein x is from 50 to 99.5 mole percent, y is from 0.5 to 50 mole percent, and z is from 0 to 10 mole percent in the defined polymer.

4. The reagent as claimed in either of claims 2 or 3 wherein the B recurring units are derived from the defined monomers wherein R is hydrogen or methyl, $R^1$ is $-CH_2CH_2X$ and L is substituted or unsubstituted phenylenealkylene or carbonyliminomethyleneiminocarbonylethylene.

5. The reagent as claimed in any of claims 1 to 4 wherein the immunological species is an antibody directed against digoxin, phenytoin, phenobarbital, thyroxine, triiodothyronine, gentamicin, carbamazepine, Primidone, tobramycin, theophylline, human chorionic gonadotropin, leutinizing hormone, a chlamydial antigen, a gonococcal antigen, a herpes antigen, a retroviral antigen or antibody or a Streptococcal antigen.

6. The reagent as claimed in any of claims 1 to 4 wherein the immunological species is a retroviral, herpes, chlamydial or gonococcal organism or antigenic component thereof, or a drug or hapten.

**7.** An element comprising an absorbent carrier material having one or more zones, the element characterized as containing in one or more of the zones a reagent as claimed in any of claims 1 to 6.

**8.** The element as claimed in claim 7 further comprising a ligand analog.

**9.** A method for the determination of an immunological ligand in an aqueous fluid comprising:
A. in the presence of an analog of the ligand, contacting a sample of the fluid with a reagent as claimed in any of claims 1 to 6,
to form an immunological complex between the immunological species and both the immunological ligand and ligand analog, and
B. determining the immunological complex as an indication of the presence of the ligand in the fluid.

**10.** The method as claimed in claim 9 wherein the ligand analog is labeled with an enzyme, and the method is carried out in the presence of reagents which will react with the enzyme to provide a detectable dye.

**11.** An agglutination method for the determination of a ligand in an aqueous liquid comprising:
A. contacting the liquid with a reagent as claimed in any of claims 1 to 6,
so as to form an agglutinate of the reaction product of the ligand and the immunological species,
B. separating the agglutinate from unagglutinated materials, and
C. determining the agglutinate as an indication of the presence of ligand in the liquid.

**12.** A method for the determination of a ligand in an aqueous liquid comprising:
A. contacting the liquid with a reagent as claimed in any of claims 1 to 6,
so as to form an insoluble immunological complex between the immunological species and the ligand,
B. prior to, simultaneously with, or subsequent to contacting step A, contacting the ligand with a second immunological species which is immunologically reactive with the ligand but which is not immunologically reactive with the first immunological species, the second species being labeled with a detectable tracer material,
so as to form a labeled insoluble complex, and
C. determining the labeled insoluble complex as an indication of the presence of the ligand in the liquid.

**13.** The method as claimed in claim 12 wherein the ligand is an antigen and the first and second immunological species are antibodies against the antigen.

**14.** The method as claimed in claim 12 wherein the ligand is an antibody, the first immunological species is an antigen reactive with the ligand, and the second immunological species is an antibody directed against the ligand.

**Patentansprüche**

**1.** Ein von oberflächenaktiven Stoffen freies Reagenz, zusammengesetzt aus:
(a) einem von oberflächenaktiven Stoffen freien polymeren Teilchen, das durch reaktive, abstehende Gruppen kovalent gebunden ist an
(b) eine immunologische Spezies, die dazu befähigt ist, an einer immunologischen Reaktion mit einem entsprechenden Rezeptor teilzunehmen,
dadurch gekennzeichnet, daß das Polymer sich von mindestens einem $\alpha,\beta$-ethylenisch ungesättigten polymerisierbaren Monoren ableitet, das entweder abstehende aktivierte 2-substituierte Ethylsulfonyl- oder Vinylsulfonylgruppen aufweist,
wobei das Innere des Teilchens von feststellbarem Tracermaterial frei ist und das Polymerteilchen hergestellt worden ist durch Emulsionspolymerisation in Abwesenheit eines oberflächenaktiven Stoffes oder eines Emulgators.

**2.** Reagenz nach Anspruch 1, in dem das Polymer durch die folgende Formel wiedergegeben wird:

$$-(A)_x-(B)_y-(D)_z-$$

worin A für wiederkehrende Einheiten steht, die sich von einem oder von mehreren hydrophoben, $\alpha,\beta$-ethylenisch ungesättigten Monomeren ableiten, wobei ferner
B für wiederkehrende Einheiten steht, die sich von einem oder von mehreren $\alpha,\beta$-ethylenisch ungesättigten Monomeren ableiten, die durch die folgende Formel wiedergegeben werden:

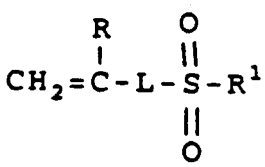

worin R für Wasserstoff steht oder eine substituierte oder unsubstituierte Alkylgruppe mit 1 bis 6 Kohlenstoffatomen,
$R^1$ für $-CH=CHR^2$ oder $-CH_2CH_2X$ steht, worin X eine abspaltbare Gruppe ist, die ersetzbar ist durch ein Nukleophil oder die in Form von HX eliminiert wird durch Behandlung mit einer Base, wobei $R^2$ für Wasserstoff steht oder eine substituierte oder unsubstituierte Alkylgruppe mit 1 bis 6 Kohlenstoffatomen oder eine substituierte oder unsubstituierte Arylgruppe mit 6 bis 12 Kohlenstoffatomen, und wobei
L eine verbindende Gruppe darstellt, die ausgewählt ist aus der Gruppe bestehend aus substituierten oder unsubstituierten Alkylengruppen mit 1 bis 20 Kohlenstoffatomen, substituierten oder unsubstituierten Arylengruppen mit 6 bis 12 Kohlenstoffatomen und einer Kombination von einer oder mehreren von jeder der Alkylen- und Arylengruppen, und wobei
D für wiederkehrende Einheiten steht, die sich von einem oder mehreren $\alpha,\beta$-ethylenisch ungesättigten Monomeren ableiten, die verschieden sind von jenen, die durch A oder B wiedergegeben werden, und wobei
x einen Zahlenwert entsprechend 0 bis 99,9 Mol-% darstellt, y ein Zahlenwert, entsprechend 0,1 bis 100 Mol-% und z ein Zahlenwert, entsprechend 0 bis 20 Mol-% .

**3.** Reagenz nach Anspruch 2, worin x für 50 bis 99,5 Mol-% steht, y für 0,5 bis 50 Mol-% und z für 0 bis 10 Mol-% in dem definierten Polymer.

**4.** Reagenz nach einem der Ansprüche 2 oder 3, in dem sich die durch B dargestellten wiederkehrenden Einheiten ableiten von den definierten Monomeren, worin R für Wasserstoff steht oder Methyl, worin $R^1$ steht für $-CH_2CH_2X$ und worin L eine substituierte oder unsubstituierte Phenylenalkylengruppe ist oder eine Carbonyliminomethyleniminocarbonylethylengruppe.

**5.** Reagenz nach einem der Ansprüche 1 bis 4, in dem die immunologische Spezies ein Antikörper ist, der gerichtet ist auf Digoxin, Phenytoin, Phenobarbital, Thyroxin, Triiodothyronin, Gentamicin, Carbamazepin, Primidon, Tobramycin, Theophyllin, menschliches chorionisches Gonadotropin, leutinisierendes Hormon, ein chlamydiales Antigen, ein gonokokkales Antigen, ein Herpesantigen, ein retrovirales Antigen oder Antikörper oder ein streptokokkales Antigen.

**6.** Reagenz nach einem der Ansprüche 1 bis 4, in dem die immunologische Spezies ein retroviraler Organismus, Herpes-, chlamydialer oder gonokokkaler Organismus oder eine antigene Komponente hiervon, oder ein Arzneimittel oder ein Hapten ist.

**7.** Element mit einem absorbierenden Trägermaterial mit einer oder mehreren Zonen, dadurch gekennzeichnet, daß es in einer oder mehreren der Zonen ein Reagenz nach einem der Ansprüche 1 bis 6 enthält.

**8.** Element nach Anspruch 7, weiter dadurch gekennzeichnet, daß es ein Liganden-Analog enthält.

**9.** Verfahren zur Bestimmung eines immunologischen Liganden in einer wäßrigen Flüssigkeit, bei dem man:

A. in Gegenwart eines Analogen des Liganden eine Probe der Flüssigkeit mit einem Reagenz gemäß einem der Ansprüche 1 bis 6 in Kontakt bringt,

um einen immunologischen Komplex zwischen der immunologischen Spezies und sowohl dem immunologischen Liganden wie auch dem Liganden-Analog zu bilden, und bei dem man

B. den immunologischen Komplex als Anzeichen für das Vorhandensein des Liganden in der Flüssigkeit bestimmt.

**10.** Verfahren nach Anspruch 9, bei dem das Liganden-Analog mit einem Enzym markiert ist, und das Verfahren in Gegenwart eines Reagenz durchgeführt wird, das mit dem Enzym unter Erzeugung eines feststellbaren Farbstoffes reagiert.

**11.** Agglutinationsmethode zur Bestimmung eines Liganden in einer wäßrigen Flüssigkeit, bei dem man:

A. die Flüssigkeit mit einem Reagenz nach einem der Ansprüche 1 bis 6 in Kontakt bringt, unter Erzeugung eines Agglutinates des Reaktionsproduktes des Liganden und der immunologischen Spezies, bei dem man

B. das Agglutinat von nicht agglutinierten Materialien trennt, und bei dem man

C. das Agglutinat als Anzeichen des Vorhandenseins des Liganden in der Flüssigkeit bestimmt.

**12.** Verfahren zur Bestimmung eines Liganden in einer wäßrigen Flüssigkeit, bei dem man:

A. die Flüssigkeit mit einem Reagenz nach einem der Ansprüche 1 bis 6 in Kontakt bringt, unter Erzeugung eines unlöslichen immunologischen Komplexes zwischen der immunologischen Spezies und dem Liganden, bei dem man

B. vor, gleichzeitig mit oder nach der Kontaktierungsstufe A, den Liganden mit einer zweiten immunologischen Spezies in Kontakt bringt, die immunologisch reaktiv mit dem Liganden ist, jedoch nicht immunologisch reaktiv mit der ersten immunologischen Spezies, wobei die zweite Spezies mit einem bestimmbaren Tracermaterial markiert ist,

um einen markierten unlöslichen Komplex zu erzeugen, und bei dem man

C. den markierten, unlöslichen Komplex als Anzeichen für das Vorhandensein des Liganden in der Flüssigkeit bestimmt.

**13.** Verfahren nach Anspruch 12, bei dem der Ligand ein Antigen ist, und bei dem die erste und die zweite immunologische Spezies Antikörper bezüglich des Antigens sind.

**14.** Verfahren nach Anspruch 12, bei dem der Ligand ein Antikörper ist, bei dem die erste immunologische Spezies ein Antigen ist, das reaktiv mit dem Liganden ist, und bei dem die zweite immunologische Spezies ein Antikörper für den Liganden ist.

## Revendications

**1.** Réactif exempt de tensioactif composé de :

(a) une particule polymérique exempte de tensioactif liée de manière covalente par des groupes pendants réactifs à

(b) une espèce immunologique qui est capable de participer à une réaction immunologique avec un récepteur correspondant,

le réactif étant caractérisé en ce que le polymère est dérivé d'au moins un monomère $\alpha,\beta$-éthyléniquement insaturé polymérisable ayant des groupes pendants activés éthylsulfonyle ou vinylsulfonyle 2-substitués,

l'intérieur de la particule étant exempt de substance traceur détectable et la particule polymérique ayant été préparée par polymérisation en émulsion en l'absence d'un tensioactif ou émulsifiant.

**2.** Réactif selon la revendication 1, dans lequel le polymère est représenté par la formule :

$$-(A)_x-(B)_y-(D)_z-$$

dans laquelle A représente des unités répétitives dérivées d'un ou plusieurs monomères $\alpha,\beta$-éthyléniquement insaturés hydrophobes,

B représente des unités répétitives dérivées d'un ou plusieurs monomères $\alpha,\beta$-éthyléniquement insaturés représentés par la formule :

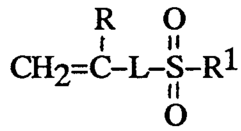

dans laquelle R est l'hydrogène ou un groupe alkyle substitué ou non substitué de 1 à 6 atomes de carbone,

$R^1$ est $-CH=CHR^2$ ou $-CH_2CH_2X$ où X est un groupe partant qui est déplacé par un nucléophile ou éliminé sous forme de HX par traitement avec une base et $R^2$ est l'hydrogène, un groupe alkyle substitué ou non substitué de 1 à 6 atomes de carbone ou un groupe aryle substitué ou non substitué de 6 à 12 atomes de carbone, et

L est un groupe de liaison choisi dans le groupe formé par les groupes alkylènes substitués ou non substitués de 1 à 20 atomes de carbone, les groupes arylènes substitués ou non substitués de 6 à 12 atomes de carbone et une combinaison d'un ou plusieurs de chacun des groupes alkylènes et arylènes,

D représente des unités répétitives dérivées d'un ou plusieurs monomères $\alpha,\beta$-éthyléniquement insaturés différents de ceux représentés par A ou B,

x est compris entre 0 et 99,9 mol %, y est compris entre 0, 1 et 100 mol % et z est compris entre 0 et 20 mol %.

**3.** Réactif selon la revendication 2, dans lequel x est compris entre 50 et 99,5 mol %, y est compris entre 0,5 et 50 mol % et z est compris entre 0 et 10 mol % dans le polymère défini.

**4.** Réactif selon l'une ou l'autre des revendications 2 ou 3, dans lequel les unités répétitives B sont dérivées des monomères définis dans lesquels R est l'hydrogène ou un groupe méthyle, $R^1$ est $-CH_2-CH_2X$ et L est un groupe phénylènealkylène ou carbonyliminométhylèneiminocarbonyléthylène substitué ou non substitué.

**5.** Réactif selon l'une quelconque des revendications 1 à 4, dans lequel l'espèce immunologique est un anticorps dirigé contre la digoxine, la phénytoïne, le phénobarbital, la thyroxine, la triiodothyronine, la gentamicine, la carbamazépine, la primidone, la tobramycine, la théophylline, la gonadotrophine chorionique humaine, l'hormone lutéinisante, un antigène chlamydial, un antigène gonococcique, un antigène herpétique, un antigène ou anticorps rétroviral ou un antigène streptococcique.

**6.** Réactif selon l'une quelconque des revendications 1 à 4, dans lequel l'espèce immunologique est un organisme rétroviral, herpétique, chlamydial ou gonococcique ou un constituant antigénique de celui-ci, ou un médicament ou haptène.

**7.** Elément comprenant un matériau support absorbant ayant une ou plusieurs zones, l'élément étant caractérisé en ce qu'il contient dans une ou plusieurs des zones un réactif selon l'une quelconque des revendications 1 à 6.

**8.** Elément selon la revendication 7, comprenant en outre un analogue de ligand.

**9.** Procédé de détermination d'un ligand immunologique dans un fluide aqueux, comprenant :

A. En présence d'un analogue du ligand, la mise en contact d'un échantillon du fluide avec un réactif selon l'une quelconque des revendications 1 à 6,

pour former un complexe immunologique entre l'espèce immunologique, le ligand immunologique et l'analogue de ligand, et

B. La détermination du complexe immunologique en tant qu'indication de la présence du ligand dans le fluide.

**10.** Procédé selon la revendication 9, dans lequel l'analogue de ligand est marqué avec une enzyme, et le procédé est mis en oeuvre en présence de réactifs qui réagissent avec l'enzyme pour former un

colorant détectable.

**11.** Procédé d'agglutination pour la détermination d'un ligand dans un liquide aqueux, comprenant :

A. la mise en contact du liquide avec un réactif selon l'une quelconque des revendications 1 à 6,

de manière à former un agglutinat du produit de la réaction du ligand et de l'espèce immunologique,

B. la séparation de l'agglutinat et des substances non agglutinées, et

C. la détermination de l'agglutinat en tant qu'indication de la présence de ligand dans le liquide.

**12.** Procédé de détermination d'un ligand dans un liquide aqueux, comprenant :

A. la mise en contact du liquide avec un réactif selon l'une quelconque des revendications 1 à 6,

de manière à former un complexe immunologique insoluble entre l'espèce immunologique et le ligand,

B. avant, pendant ou après l'étape de mise en contact A, la mise en contact du ligand avec une seconde espèce immunologique qui est immunologiquement réactive avec le ligand mais qui n'est pas immunologiquement réactive avec la première espèce immunologique, la seconde espèce étant marquée avec une substance traceur détectable,

de manière à former un complexe insoluble marqué, et

C. la détermination du complexe insoluble marqué en tant qu'indication de la présence du ligand dans le liquide.

**13.** Procédé selon la revendication 12, dans lequel le ligand est un antigène et les première et seconde espèces immunologiques sont des anticorps contre l'antigène.

**14.** Procédé selon la revendication 12, dans lequel le ligand est un anticorps, la première espèce immunologique est un antigène réactif avec le ligand et la seconde espèce immunologique est un anticorps dirigé contre le ligand.